(19) Europäisches Patentamt
European Patent Office
Office européen des brevets

(11) Veröffentlichungsnummer: **0 306 696 B1**

(12) **EUROPÄISCHE PATENTSCHRIFT**

(45) Veröffentlichungstag der Patentschrift: **16.02.94**

(21) Anmeldenummer: **88112421.8**

(22) Anmeldetag: **01.08.88**

(51) Int. Cl.$^5$: **C07D 401/06**, C07D 213/40, C07C 279/28, C07D 295/20, A01N 43/40, A01N 43/50, A01N 43/54

# (54) Substituierte Guanidine.

(30) Priorität: **04.08.87 CH 2985/87**
**06.06.88 CH 2141/88**

(43) Veröffentlichungstag der Anmeldung:
**15.03.89 Patentblatt 89/11**

(45) Bekanntmachung des Hinweises auf die Patenterteilung:
**16.02.94 Patentblatt 94/07**

(84) Benannte Vertragsstaaten:
**CH DE FR GB IT LI**

(56) Entgegenhaltungen:

| | |
|---|---|
| EP-A- 0 126 558 | EP-A- 0 154 178 |
| EP-A- 0 163 855 | EP-A- 0 235 725 |
| WO-A-83/00627 | DE-A- 3 148 103 |
| DE-B- 1 443 913 | US-A- 2 697 727 |
| US-A- 3 074 955 | US-A- 4 098 791 |

**JOURNAL OF MEDICINIAL CHEMISTRY, Band 21, Nr. 8, 1978, Seiten 773-781 ; C.K. NIELSEN et al.: "Synthesis amd hypotensive activity of N-alkyl-N"-cyano-N'-pyridylguanidines"**

(73) Patentinhaber: **CIBA-GEIGY AG**
**Klybeckstrasse 141**
**CH-4002 Basel(CH)**

(72) Erfinder: **Gsell, Laurenz, Dr.**
**Maiengasse 56**
**CH-4056 Basel(CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 306 696 B1

JOURNAL OF HETEROCYCLIC CHEMISTRY, Band 23, Nr. 2, März-April 1986, Seiten 401-408 ; J. REITER et al.: "On triazoles. V(1,2). Synthesis of 1- and 2-R1-3-R2,R3-amino-5-amino-1,2,4-triazoles"

CHEMICAL ABSTRACTS, Band 85, Nr. 21, 22. November 1976, Columbus, Ohio, US, Seite 489, Spalte 2, Abstract Nr. 159372z

JOURNAL FUER PRAKTISCHE CHEMIE, Band 318, Heft 3, 1976, Seiten 479-482 ; G. REMBARZ et al. : "Darstellung von substituierten 1.2.4-Oxadiazolen aus N-Cyanverbindungen"

ARCHIVE DER PHARMAZIE, Band 310, Nr. 10, 1977, Seiten 820-827 ; R. NEIDLEIN et al : "Zum Reaktionsverhalten von Isonitrildichloriden, Iminothiokohlensäureesterchloriden und Bis-methylmercaptomethylencyanamid gegeüber Enaminen sowie Oximen"

ARCHIVE DER PHARMAZIE, Band 320, Nr. 7, 1987, Seiten 608-616 ; J. BONJEAN et al. : "Isolamtidin und Analoge"

JOURNAL FUER PRAKTISCHE CHEMIE, Band 323, Nr. 4, 1981, Seiten 694-699 ; D. MARTIN et al. : "Einfache Synthesen für Amino-aroxy-1,3,5-triazine"

ARZENEIMITTELFORSCHUNG, Band 29(I), Nr. 4, November 1979, Seiten 595-598 ; W. SCHUNACK et al. : "Synthese und H2-antihistaminische Wirkung ringmethylierter Metiamid-Analoga"

YUKI GOSEI KAGAKU KYOKAI SHI, Band 29, Nr. 1, 1971, Seiten 65-73 ; T. SUYAMA et al.: "Synthetic study of cyanamide-dithiocarbonic acid ester and related compounds (Part1)"

CHEMISCHE BERICHTE, Band 100, 1967, Seiten 2609-2615 ; E. ALLENSTEIN et al.: "Ueber de N-Cyan-imidokohlensäure-diäthylester und O-Aethyl-N-cyan-isoharnstoffe"

CHEMISCHE BERICHTE, Band 101, 1968, Seiten 3185-3200 ; D. MARTIN et al.: "Cyansäureester, XVI: Spaltung der Si-N-Bindung durch Cyansäureester, Synthese von N-Trimethylsilyl- und N-Cyan-isoharnstoffen"

CHEMICAL ABSTRACTS, Band 90, Nr. 11, 12. März 1979, Columbus, Ohio, US, Seite 608, Spalte 2, Abstract Nr. 87289f ; y. HIRATA et al.:"Guanidines"

## Beschreibung

Die vorliegende Erfindung betrifft neue, substituierte N-Pyridylmethyl-N'-cyano-guanidine, Verfahren zu ihrer Herstellung und ihre Verwendung in der Schädlingsbekämpfung.

Die Erfindung bezieht sich auf neue Verbindungen der Formel I und gegebenenfalls deren Tautomere

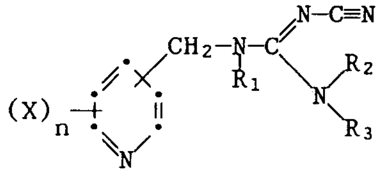

(I),

worin

$R_1$ Wasserstoff, $C_1$-$C_4$-Alkyl oder Cyclopropyl;
$R_2$ Wasserstoff, $C_1$-$C_4$-Alkyl, Cyclopropyl, Benzyl oder Picolyl;
$R_3$ Wasserstoff, $C_1$-$C_4$-Alkyl oder Cyclopropyl; oder $R_2$ und $R_3$ zusammen $-(CH_2)_4-$ oder $-(CH_2)_5-$;
X Halogen; und
n 0, 1, 2 oder 3

bedeuten, sowie die Salze von Verbindungen der Formel I; mit Ausnahme von N-Picolyl-N-methyl-N'-cyanoguanidin, N-Picolyl-N'-methyl-N''-cyanoguanidin, N-Picolyl-N'-cyanoguanidin, N-(2-Picolyl)-N'-t-butyl-N''-cyanoguanidin und von N-(3-Picolyl)-N'-t-butyl-N''-cyanoguanidin.

Bevorzugt werden erfindungsgemäss Verbindungen der Formel I und gegebenenfalls deren Tautomere, worin $R_1$ und $R_3$ Wasserstoff oder Methyl;
$R_2$ Wasserstoff, $C_1$-$C_4$-Alkyl oder Benzyl; oder
$R_2$ und $R_3$ zusammen den Rest $-(CH_2)_5-$;
X Chlor und
n 0, 1, oder 2

bedeuten, sowie Salze von Verbindungen der Formel I.

Hervorzuheben sind insbesondere Verbindungen der Formel I und gegebenenfalls deren Tautomere, worin n für 0 steht, sowie Salze von Verbindungen der Formel I.

Weiterhin bevorzugt werden solche erfindungsgemässen Verbindungen der Formel I und gegebenenfalls deren Tautomere, worin

$R_1$ Wasserstoff oder Methyl;
$R_2$ Wasserstoff oder $C_1$-$C_4$-Alkyl;
$R_3$ Wasserstoff, Methyl oder Aethyl; und
n die Zahl O

bedeuten; sowie Salze von Verbindungen der Formel I.

Unter dem Begriff Alkyl selbst oder als Bestandteil eines anderen Substituenten sind geradkettige oder verzweigte und je nach Zahl der angegebenen Kohlenstoffatome im Rahmen der vorliegenden Erfindung beispielsweise folgende Gruppen zu verstehen: Methyl, Ethyl, Propyl, Butyl, sowie ihre Isomeren, wie Isopropyl, Cyclopropyl, Isobutyl, tert.-Butyl, sek.-Butyl.

Unter dem Begriff Halogen im Rahmen der vorliegenden Erfindung ist Fluor, Chlor und Brom zu verstehen, vorzugsweise Fluor und Chlor.

Die vorliegende Erfindung umfasst bei Verbindungen der Formel I, in denen $R_2$ oder $R_3$ Wasserstoff bedeuten, auch deren Tautomere:

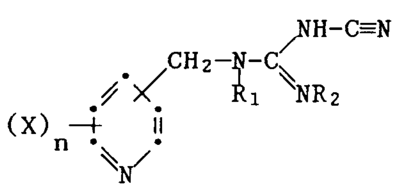

bzw.

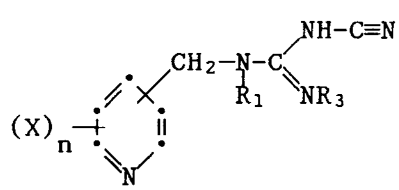

.

Gegenstand der vorliegenden Erfindung sind ferner die Salze, insbesondere die nicht-toxischen, pflanzenphysiologisch unbedenklichen Salze, der Verbindungen der Formel I. Als derartige Salze mit

organischen und anorganischen Säuren kommen z.B. die folgenden in Betracht: Chloride, Bromide, Jodide, Sulfate, Hydrogensulfate, Chlorate, Perchlorate, Rhodanide, Nitrate, Phosphate, Hydrogenphosphate, Tetrafluorborate, Formiate, Acetate, Trichloracetate, Trifluoracetate, Phenylsulfonate, Oxalate, Malonate, Succinate, Malate, Tartrate oder Citrate.

Die Verbindungen der Formel I sowie ihre Salze können hergestellt werden, indem man

a) eine Verbindung der Formel II

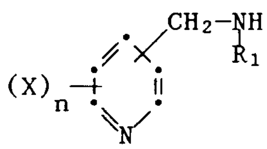

(II)

mit einer Verbindung der Formel III

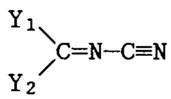

(III)

und einer Verbindung der Formel IV

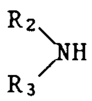

(IV)

umsetzt; oder

b) zur Herstellung einer Verbindung der Formel I, worin $R_2$ und $R_3$ Wasserstoff bedeuten, eine Verbindung der Formel II mit Dicyanimid umsetzt;

wobei in den vorstehenden Formeln II bis IV

$R_1$, $R_2$ und $R_3$

sowie X und n die in Anspruch 1 angegebenen Bedeutungen haben und

$Y_1$ und $Y_2$ abspaltbare Abgangsgruppen bedeuten;

und indem man erwünschtenfalls eine nach a) oder b) erhaltene Verbindung der Formel I in an sich bekannter Weise in eines ihrer Salze umwandelt.

Die obigen Verfahrensvarianten a) und b) können auch in der Weise durchgeführt werden, dass man zunächst eine Verbindung der Formel III mit einer Verbindung der Formel IV unter Bildung eines Zwischenproduktes der Formel VI

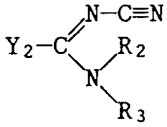

(VI)

umsetzt und das erhaltene Zwischenprodukt der Formel VI anschliessend mit einer Verbindung der Formel II umsetzt, wobei $R_1$, $R_2$, $R_3$, X, n, $Y_1$ und $Y_2$ die in Anspruch 6 angegebenen Bedeutungen haben.

Im Rahmen der vorstehenden Verfahrensvarianten kommen als abspaltbare Abgangsgruppen in Verbindungen der Formeln III und VI z.B. die folgenden in Betracht: $-S-CH_3$, $-O-C_2H_5$,

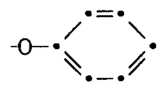 .

Die zu den erfindungsgemässen Verbindungen der Formel I führenden obigen Verfahrensvarianten werden vorzugsweise in einem Lösungsmittel vorgenommen. Geeignete Lösungsmittel sind z.B. aromatische Kohlenwasserstoffe, wie Benzol, Toluol und Xylol; Ketone, wie Aceton, Cyclohexanon und Methyläthylketon; Aether, wie Tetrahydrofuran, Dioxan und Diäthyläther; halogenierte Kohlenwasserstoffe, wie Chloroform, Tetrachlorkohlenstoff und Chlorbenzol; Alkohole, wie Aethanol und Propanol; Ester aliphatischer Säuren, wie Aethylacetat; aliphatische Amide, wie Dimethylformamid und Dimethylacetamid; Dimethylsulfoxid, Acetonitril und andere Lösungsmittel, die die Reaktion nicht beeinträchtigen. Diese Lösungsmittel können auch als Gemische verwendet werden. Die Reaktionstemperatur kann in einem weiten Bereich von -10 bis +150 °C liegen. Bevorzugt wird ein Temperaturbereich von etwa 20 ° bis 80 °C.

Die oben beschriebenen Arbeitsweisen zur Herstellung der Verbindungen der Formel I sind an sich bekannt und werden im Prinzip in den EP-Patentanmeldungen Nr. 2930 und 161.841, der US-Patentschrift Nr. 2.455.807 und der DE-OS Nr. 340.980 aufgezeigt.

Die Ausgangsverbindungen der Formeln II, III und IV sind bekannt oder können, falls sie neu sind, analog bekannten Methoden erhalten werden. So sind die N-Cyanoiminocarbonate der Formel III sowie ihre Herstellung aus Chem. Ber. 100, 2604-15 (1967); J. Hetero. Chem. 19, 1205-6 (1982), Arch. Pharm. 318, 888 (1985) und der JP-Patentanmeldung SHO 61-76044 bekannt. Die Picolylamin-Verbindungen der Formel II sind bekannt oder analog bekannten Verfahren zugänglich [vgl. Tetrahedron Letters 26, 5863 (1985)].

Bestimmte N-Picolyl-N-methyl-N'-cyano-guanidine, ihre Herstellung sowie ihre Verwendung als Zwischenprodukte für die Synthese von Pharmazeutika sind bereits in den US-Patentschriften Nr. 3.074.955 und Nr. 3.147.271 beschrieben worden. Die Herstellung von pharmazeutisch wirksamen N-Picolyl-N'-methyl-N''-cyano-guanidinen wird auch in C.A. Vol. 90 (1979) 90:87289 f erwähnt. N-Picolyl-N'-cyanoguanidine werden in WO-83/00627, N-Picolyl-N'-t-butyl-N''-cyanoguanidine in J. Med. Chem. 21, 779 (1978) genannt.

Es wurde nun überraschenderweise gefunden, dass die erfindungsgemässen neuartigen Guanidin-Verbindungen der Formel I bei guter Pflanzenverträglichkeit und geringer Warmblütertoxizität ausgezeichnete Wirksamkeit als Schädlingsbekämpfungsmittel aufweisen.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen entspricht einer Abtötungsrate (Mortalität) von mindestens 50-60 % der erwähnten Schädlinge.

Mit Hilfe der erfindungsgemässen Verbindungen der Formel I können vor allem pflanzenschädigende Insekten, speziell pflanzenschädigende Insekten in Zier- und Nutzpflanzungen, insbesondere in Baumwollkulturen, Gemüsekulturen, Reiskulturen und Obstkulturen, bekämpft werden. In diesem Zusammenhang ist hervorzuheben, dass die genannten Verbindungen sich sowohl durch eine stark ausgeprägte systemische, vor allem aber durch Kontakt-Wirkung gegen saugende Insekten, insbesondere gegen Insekten der Familie Aphididae (wie z.B. Aphis fabae, Aphis craccivora und Myzus persicae), welche sich mit herkömmlichen Mitteln nur schwierig bekämpfen lassen, auszeichnen.

Die Verbindungen der Formel I zeichnen sich weiterhin durch eine gute Wirkung gegen larvale Insektenstadien und Nymphen, speziell von fressenden Schadinsekten, aus. Insbesondere können die Verbindungen der Formel I mit ausgezeichnetem Erfolg gegen pflanzenschädigende Zikaden, speziell in Reis-Kulturen, eingesetzt werden. In diesem Zusammenhang wird auf die geringe Fisch-Toxizität der erfindungsgemässen Verbindungen hingewiesen.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise

verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel 1 oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäureoder eines Naphthalinsulfonsäure-Formaldehydkondensationsproduktes. Ferner kommen auch entsprechende Phpsphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"MC cutcheon's Detergents and Emulsifiers Annual"

MC Publishing Corp., Ridgewood, New Jersey, 1979;

Dr. Helmut Stache "Tensid Taschenbuch", Carl Hanser Verlag München/Wien 1981.

Die pestiziden Zubereitungen enthalten - auf das Gewicht bezogen - in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen, z.B. 0,1 bis 1000 ppm.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung von N-Pyrid-3-ylmethyl-N'-äthyl-N''-cyanoguanidin

Es werden 6,2 g N-Pyrid-3-ylmethyl-N′-cyano-S-methylisothioharnstoff zusammen mit 30 ml Aethylamin (70%-ig in Wasser) während einer Stunde bei 60 °C verrührt und anschliessend für 30 Minuten auf 80 °C erhitzt. Das beim Abkühlen des Ansatzes auskristallisierende Produkt wird mit kaltem Wasser gewaschen und entspricht der Titelverbindung der Formel

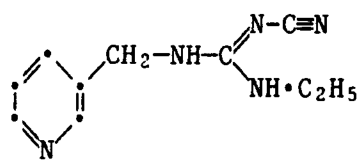

mit einem Smp. von 177-179 °C (Verbindung Nr. 1).

Beispiel 2: Herstellung von N-Pyrid-3-ylmethyl-N'-methyl-N'-n-butyl-N''-cyanoguanidin

Es werden 7,31 g Dimethyl-N-cyanothioiminocarbonat, 4,79 g n-Butylmethylamin (in 20 ml Acetonitril) und 20 mg Dimethylaminopyridin zusammen während einer Stunde bei 70 °C gehalten. Nach Entfernen des Lösungsmittels wird der N,S-Dimethyl-N-n-butyl-N'-cyanoisothioharnstoff in Form eines Oels erhalten. Dieses Oel wird zusammen mit 6,41 g 3-Picolylamin und 5,5 g 1,4-Diazabicyclo[2.2.2]oktan (in 30 ml Toluol) während 30 Stunden am Rückfluss gehalten. Die Lösungsmittel werden dann entfernt, der Rückstand wird in Dichlormethan aufgenommen und die Lösung nacheinander mit $H_2O$, 2n NaOH-Lösung und gesättigter Kochsalzlösung je zweimal gewaschen. Die Dichlormethan-Lösung wird über $Na_2SO_4$ getrocknet und eingeengt. Das als Rückstand zurückbleibende Rohprodukt wird an Kieselgel mittels Dichlormethan/Methanol (0-10 %) chromatographiert und aus Aceton/Aether umkristallisiert. Auf diese Weise erhält man die Titelverbindung der Formel

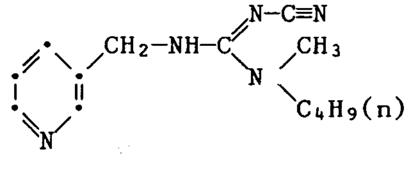

mit einem Smp. von 65-69 °C (Verbindung Nr. 2).

Beispiel 3:

Wie vorstehend angegeben werden die folgenden Verbindungen der Formel I hergestellt:

| Verbindung Nr. | Stellung Pyridyl | $R_1$ | $R_2$ | $R_3$ | n | X | physikal. Daten |
|---|---|---|---|---|---|---|---|
| 3 | 3- | $-CH_3$ | $-C_4H_9(n)$ | H | 0 | - | $n_D^{22}$ = 1,5595 |
| 4 | 3- | H | $-C_4H_9(n)$ | H | 0 | - | F = 87-90°C |
| 5 | 3- | H | $-CH_2-$ 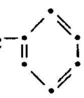 | H | 0 | - | F = 178-180°C |
| 6 | 3- | H | $-(CH_2)_4-$ | | 0 | - | F = 151-153°C |
| 7 | 4- | H | $-CH_2CH_3$ | H | 0 | - | F = 150-152°C |
| 8 | 4- | H | $-C_4H_9(n)$ | H | 0 | - | F = 126-129°C |
| 9 | 4- | H | $-(CH_2)_3-$ | | 0 | - | F = 160-163° |
| 10 | 4- | H | $-CH_2-$ 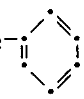 | H | 0 | - | F = 153-156°C |
| 11 | 3- | $-CH_3$ | $-C_3H_7(i)$ | H | 0 | - | dickflüssiges Oel |
| 12 | 3- | H | $-CH_3$ | $-CH_3$ | 0 | - | F = 70,5-75,5°C |
| 13 | 3- | H | $-C_3H_7(i)$ | H | 0 | - | F = 164-165,5°C |
| 14 | 3- | H | $-C_2H_5$ | $-C_2H_5$ | 0 | - | dickflüssiges Oel |
| 15 | 3- | H | $-C_4H_9(i)$ | H | 0 | - | F = 125,5-127°C |
| 16 | 3- | H | $-CH_2-$ 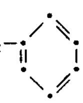 | $-CH_3$ | 0 | - | F = 123-126°C |
| 17 | 3- | $-CH_3$ | $-C_4H_9(i)$ | H | 0 | - | dickflüssiges Oel |
| 18 | 4- | H | $-CH_3$ | $-CH_3$ | 0 | - | F = 121-122,5°C |
| 19 | 4- | H | H | $-C_3H_7(i)$ | 0 | - | F = 159-161°C |
| 20 | 4- | H | $-CH_3$ | $-C_4H_9(n)$ | 0 | - | F=130,5-137,5°C |
| 21 | 3- | $-CH_3$ | $-CH_3$ | $-CH_3$ | 0 | - | $n_D^{25}$ = 1,5772 |

| Verbindung Nr. | Stellung Pyridyl | $R_1$ | $R_2$ | $R_3$ | n | X | physikal. Daten |
|---|---|---|---|---|---|---|---|
| 22A | 4- | $-C_3H_7(n)$ | H | $-CH_3$ | 0 | - | Harz (Tautomer A) |
| 22B | 4- | $-C_3H_7(n)$ | H | $-CH_3$ | 0 | - | Harz (Tautomer B) |
| 23 | 3- | $-C_4H_9(n)$ | H | $-CH_3$ | 0 | - | Harz |
| 24A | 4- | $-C_3H_7(n)$ | H | $-C_3H_7(n)$ | 0 | - | Harz (Tautomer A) |
| 24B | 4- | $-C_3H_7(n)$ | H | $-C_3H_7(n)$ | 0 | - | Harz (Tautomer B) |
| 25 | 2- | H | H | $-C_3H_7(i)$ | 0 | - | F = 125,5-127°C |
| 26 | 2- | H | $-CH_3$ | $-C_4H_9(n)$ | 0 | - | $n_D^{24}$ = 1,559 |

| Stellung Pyridyl*) | $R_1$ | $R_2$ | $R_3$ |
|---|---|---|---|
| 3- | $-C_2H_5$ | $-C_3H_7(i)$ | H |
| 4- | H | $-C_3H_7(i)$ | H |
| 3- | H | $-C_4H_9(s)$ | H |
| 4- | H | $-C_2H_5$ | $-C_2H_5$ |
| 3- | H | $-CH_3$ | $-C_2H_5$ |
| 3- | H | $-CH_3$ | $-C_4H_9(n)$ |
| 3- | $-CH_3$ | $-CH_2-$ 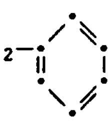 | H |
| 3- | 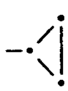 | $-CH_3$ | H |
| 3- | 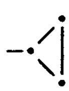 | $-C_3H_7(i)$ | H |
| 3- | $-CH_3$ | 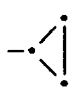 | H |

*) n=0

Beispiel 4:

Formulierungen für Wirkstoffe der Formel I gemäss Beispiel 1-3, resp. Kombinationen dieser Wirkstoffe mit andern Insektiziden oder Akariziden (% = Gewichtsprozent):

1. Spritzpulver

| | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

3. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 5: Wirkung gegen Lucilia sericata

Zu 9 ml eines Zuchtmediums wird bei 50°C ein ml einer 0,1 % Aktivsubstanz enthaltenden wässrigen Zubereitung hinzugefügt. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1-3 zeigen in diesem Test gute Wirkung (Mortalität) gegen Lucilia sericata.

Beispiel 6: Wirkung gegen Aëdes aegypti

Auf die Oberfläche von 150 ml Wasser, das sich in einem Behälter befindet, wird so viel einer 0,1%igen acetonischen Lösung des Wirkstoffs pipettiert, dass eine Konzentration von 400 ppm erhalten wird. Nach Verdunsten des Acetons wird der Behälter mit 30 bis 40 2-tägigen Aëdes-Larven beschickt. Nach 2 und 7 Tagen wird die % Mortalität (Anzahl der schwimmunfähigen Larven) geprüft.

Verbindungen der Formel I gemäss Beispiel 1-3 zeigen gute Wirkung (Mortalität) im obigen Test.

Beispiel 7: Insektizide Kontaktwirkung: Aphis craccivora

In Töpfen gezogene Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe besprüht. Man verwendet pro Test-Verbindung zwei Pflanzen, und eine Auswertung der erzielten Abtötungsrate erfolgt nach weiteren 24 Stunden.

Verbindungen der Formel I gemäss Beispiel 1-3 zeigen gute Wirksamkeit (Mortalität) in diesem Test.

Beispiel 8: Insektizide systemische Wirkung: Aphis craccivora

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25%igen Spritzpulver) jeweils in einer Konzentration von 400 ppm direkt auf de Erde in den Töpfen.

Nach 24 Stunden werden auf die oberirdischen Pflanzenteile Läuse der Spezies Aphis craccivora gesetzt und die Pflanzen mit einem Plastikzylinder überstülpt, um die Läuse vor einer eventuellen Kontakt- oder Gaswirkung der Testsubstanz zu schützen.

Die Auswertung der erzielten Abtötung erfolgt 48 und 72 Stunden nach Versuchsbeginn. Pro Testsubstanz werden zwei Pflanzen, je eine in einem separaten Topf, verwendet. Der Versuch wird bei 25 °C und 70 % relativer Luftfeuchtigkeit durchgeführt.

Die Verbindungen der Formel I gemäss Beispiel 1-3 zeigen gute Wirkung in obigem Test.

Beispiel 9: Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 400 ppm der zu prüfenden Verbindung bis zur Tropfnässe besprüht. Man verwendet pro Ansatz zwei Pflanzen. Eine Auswertung der erzielten Abtötungsrate erfolgt 48 Stunden nach Applikation. Der Versuch wird bei 20-22 °C und 60 % relativer Luftfeuchtigkeit durchgeführt.

Die Verbindungen der Formel I gemäss Beispiel 1-3 zeigen gute Wirkung in obigem Test.

Beispiel 10: Insektizide systemische Wirkung: Myzus persicae

Bewurzelte Kohlpflanzen im 4- bis 5-Blatt-Stadium werden in Töpfe, welche 60 ccm Erde enthalten, verpflanzt. Anschliessend werden 50 ml einer wässrigen Formulierung der zu prüfenden Verbindung I (erhalten aus einem 25%igen Spritzpulver) jeweils in einer Konzentration von 400 ppm direkt auf die Erde aufgegossen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen Kontakt- oder Gaswirkung der Testsubstanz zu schützen.

Die Auswertung der erzielten %-Abtötung erfolgt 48 Stunden nach Versuchsbeginn. Pro Testsubstanz werden zwei Pflanzen, je eine in separaten Töpfen, verwendet. Der Versuch wird bei ca. 25 °C und 60 % relativer Luftfeuchtigkeit durchgeführt.

Die Verbindungen der Formel I gemäss Beispiel 1-3 zeigen gute Wirkung in obigem Test.

Beispiel 11: Insektizide Blattpenetrations-Wirkung: Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffs in einer Konzentration von 400 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende

Blattläuse abzutöten.

Der Versuch wird bei etwa 20 °C und 60 % relativer Luftfeuchtigkeit durchgeführt. Die Auswertung auf %-Mortalität erfolgt 48 Stunden nach Wirkstoffapplikation.

Verbindungen der Formel I gemäss Beispiel 1-3 zeigen gute Wirkung in obigem Test.

Beispiel 12: Insektizide Wirkung (systemisch-Wasser): Aphis craccivora

Erbsenkeimlinge, die 24 Stunden vor Beginn des Versuches mit den Blattläusen infestiert worden waren, werden in 20 ml einer wässrigen Brühe gestellt, die 400 ppm des zu prüfenden Wirkstoffes enthält. Die wässrige Brühe wird aus einem Emulsionskonzentrat oder einer benetzbaren Pulverzubereitung des betreffenden Wirkstoffes hergestellt und befindet sich in einem Gefäss, dass mit einem Löcher aufweisenden Plastikdeckel abgeschlossen ist. Die Wurzel der infestierten Erbsenpflanze wird jeweils durch ein Loch in dem Plastikdeckel in die Brühe geschoben. Das Loch wird dann mit Watte abgedichtet, um die Pflanze zu fixieren und einen eventuellen Einfluss der Gasphase aus der Brühe auszuschalten.

Der Versuch wird bei 20 °C und 60 % relativer Luftfeuchtigkeit durchgeführt. Nach zwei Tagen wird auf die Anzahl der nicht mehr saugfähigen Testtiere im Vergleich zu unbehandelten Kontrollen bonitiert. Damit wird festgestellt, ob der über die Wurzel aufgenommene Wirkstoff, die Blattläuse an den oberen Pflanzenteilen abtötet.

Verbindungen der Formel I gemäss Beispiel 1-3 zeigen im obigen Versuch eine gute systemische Wirkung gegen Insekten der Spezies Aphis craccivora.

Beispiel 13: Frassgift- und Kontakt-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Formel I gemäss Beispiel 1-3 zeigen gute Wirkung in diesem Test gegen Nilaparvata lugens.

Beispiel 14: Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 400 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wurde dann mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N 2 bis N 3 Stadium besiedelt und mit einem Plastikzylinder abgedeckt. Der Versuch wird bei 20 °C und 60 % relativer Luftfeuchtigkeit mit einer Beleuchtungsperiode von 16 Stunden durchgeführt. Nach fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen der Formel I gemäss Beispiel 1-3 zeigen im obigem Test gute Wirkung (Mortalität) gegen Nilaparvata lugens.

Beispiel 15: Insektizide Frassgift- und Kontakt-Wirkung

Ca. 25 cm hohe eingetopfte Baumwollpflanzen werden mit wässrigen Wirkstoffemulsionen besprüht, die den Wirkstoff in einer Konzentration von 800 ppm enthalten.

Nach dem Antrocknen des Sprühbelages werden die Baumwollpflanzen mit Spodoptera littoralis- bzw. Heliothis virescens-Larven im ersten larvalen Stadium besiedelt. Der Versuch wird bei 24 °C und etwa 60 % relativer Luftfeuchtigkeit durchgeführt. Nach 120 Stunden wird die %-Mortalität der Test-Insekten gegenüber

unbehandelten Kontrollen bestimmt.

Verbindungen der Formel I gemäss Beispiel 1-3 zeigen gute Wirkung (Mortalität) in diesem Test.

Beispiel 16: Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23 °C, bei 55 % relativer Luftfeuchtigkeit und mit einer Belichtungsperiode von 16 Stunden durchgeführt.

Verbindungen der Formel I gemäss Beispiel 1-3 zeigen in diesem Test gute Wirkung.

## Patentansprüche

**1.** Verbindung der Formel I und gegebenenfalls deren Tautomere

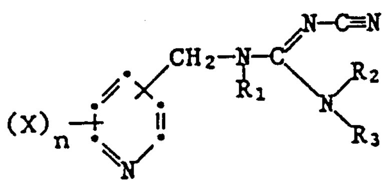

(I),

worin

$R_1$ Wasserstoff, $C_1$-$C_4$-Alkyl oder Cyclopropyl;

$R_2$ Wasserstoff, $C_1$-$C_4$-Alkyl, Cyclopropyl, Benzyl oder Picolyl;

$R_3$ Wasserstoff, $C_1$-$C_4$-Alkyl, oder Cyclopropyl; oder $R_2$ und $R_3$ zusammen $-(CH_2)_4-$ oder $-(CH_2)_5-$;

X Halogen; und

n eine Zahl 0, 1, 2 oder 3

bedeuten, sowie die Salze von Verbindungen der Formel I; mit Ausnahme von N-Picolyl-N-methyl-N'-cyanoguanidin, von N-Picolyl-N'-methyl-N''-cyanoguanidin, von N-Picolyl-N'-cyanoguanidin,von N-(2-Picolyl)-N'-t-butyl-N''-cyanoguanidin und von N-(3-Picolyl)-N'-t-butyl-N''-cyanoguanidin.

**2.** Verbindung der Formel I und ggf. deren Tautomere gemäß Anspruch 1, dadurch gekennzeichnet, daß

$R_1$ und $R_3$ Wasserstoff oder Methyl;

$R_2$ Wasserstoff, $C_1$-$C_4$-Alkyl oder Benzyl; oder $R_2$ und $R_3$ zusammen $-(CH_2)_5-$

X Chlor und n 0,1 oder 2 bedeuten, sowie die Salze von Verbindungen der Formel I.

**3.** Verbindung der Formel I und gegebenenfalls deren Tautomere gemäß Anspruch 1 oder 2, dadurch gekennzeichnet, daß n die Zahl 0 bedeutet, sowie die Salze von Verbindungen der Formel I.

**4.** Verbindung der Formel I und gegebenenfalls deren Tautomeren gemäß Anspruch 1, dadurch gekennzeichnet, daß

$R_1$ Wasserstoff oder Methyl;

$R_2$ Wasserstoff oder $C_1$-$C_4$-Alkyl;

$R_3$ Wasserstoff, Methyl oder Aethyl und n 0 bedeuten, sowie die Salze von Verbindungen der Formel I.

5. Verbindung gemäß Anspruch 4 der Formel

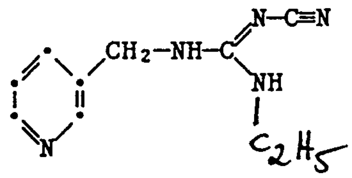 .

6. Verfahren zur Herstellung einer Verbindung der Formel I oder gegebenenfalls eines Tautomeren davon gemäß Anspruch 1 oder eines Salzes einer solchen Verbindung I, dadurch gekennzeichnet, daß man

a) eine Verbindung der Formel II

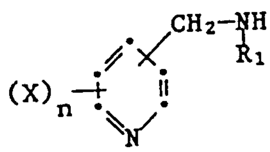 (II)

mit einer Verbindung der Formel III

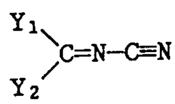 (III)

und einer Verbindung der Formel IV

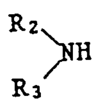 (IV)

umsetzt; oder

b) zur Herstellung einer Verbindung der Formel I, worin $R_2$ und $R_3$ Wasserstoff bedeuten, eine Verbindung der Formel II mit Dicyanimid umsetzt;

wobei in den vorstehenden Formeln II bis IV $R_1$, $R_2$ und $R_3$ sowie X und n die in Anspruch 1 angegebenen Bedeutungen haben und $Y_1$ und $Y_2$ abspaltbare Abgangsgruppen bedeuten;

und daß man erwünschtenfalls eine nach a) oder b) erhaltene Verbindung der Formel I in an sich bekannter Weise in eines ihrer Salze überführt.

7. Verfahren gemäß Anspruch 6a) oder 6b), dadurch gekennzeichnet, daß man eine Verbindung der Formel III mit einer Verbindung der Formel IV unter Bildung eines Zwischenprodukts der Formel VI

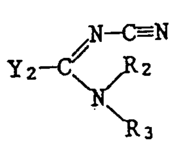 (VI)

umsetzt und das erhaltene Zwischenprodukt der Formel VI anschließend mit einer Verbindung der Formel II umsetzt, wobei $R_1$, $R_2$, $R_3$, X, n, $Y_1$ und $Y_2$ die in Anspruch 6 angegebene Bedeutung haben.

**8.** Verwendung einer Verbindung der Formel I oder gegebenenfalls eines Tautomeren davon gemaß Anspruch 1 oder eines Salzes einer solchen Verbindung der Formel I zur Bekämpfung von Pflanzen befallenden Insekten und Vertretern der Ordnung Akarina.

**9.** Verwendung gemäß Anspruch 8 zur Bekämpfung von fressenden Schadinsekten in Reiskulturen.

**10.** Mittel zur Bekämpfung von Pflanzen befallenden Schadinsekten und Schädlingen der Ordnung Akarina, enthaltend als aktive Komponente mindestens eine Verbindung der Formel I oder gegebenenfalls ein Tautomeres davon gemäß Anspruch 1 oder ein Salz einer solchen Verbindung I.

**11.** Verfahren zur Bekämpfung von Pflanzen befallenden Insekten und Vertretern der Ordnung Akarina, dadurch gekennzeichnet, daß man die Schädlinge bzw. deren verschiedene Entwicklungsstadien oder deren Aufenthaltsort mit einer pestizid wirksamen Menge einer Verbindung der Formel I oder gegebenenfalls eines Tautomeren davon gemäß Anspruch 1 oder eines Salzes einer solchen Verbindung I oder mit einem Mittel gemäß Anspruch 10, enthaltend neben Zusatz- und Trägerstoffen eine pestizid wirksame Menge der aktiven Komponente, in Kontakt bringt oder behandelt.

**Claims**

**1.** A compound of formula I and, where applicable, tautomers thereof

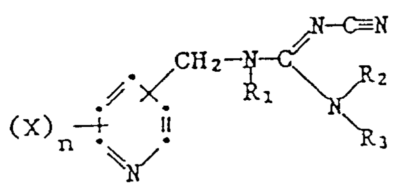

(I)

in which
$R_1$ is hydrogen, $C_1$-$C_4$alkyl or cyclopropyl;
$R_2$ is hydrogen, $C_1$-$C_4$alkyl, cyclopropyl, benzyl or picolyl;
$R_3$ is hydrogen, $C_1$-$C_4$alkyl or cyclopropyl;
or $R_2$ and $R_3$ together are $-(CH_2)_4-$ or $-(CH_2)_5-$;
X is halogen; and
n is an integer 0, 1, 2 or 3;
or a salt of a compound of formula I; with the exception of N-picolyl-N-methyl-N'-cyanoguanidine, N-picolyl-N'-methyl-N''-cyanoguanidine, N-picolyl-N'-cyanoguanidine, N-(2-picolyl)-N'-t-butyl-N''-cyanoguanidine and N-(3-picolyl)-N'-t-butyl-N''-cyanoguanidine.

**2.** A compound of formula I and, where applicable, tautomers thereof according to claim 1, wherein
$R_1$ and $R_3$ are hydrogen or methyl;
$R_2$ is hydrogen, $C_1$-$C_4$alkyl or benzyl;
or $R_2$ and $R_3$ together are $-(CH_2)_5-$;
X is chlorine; and
n is 0, 1 or 2,
or a salt of a compound of formula I.

**3.** A compound of formula I and, where applicable, tautomers thereof according to claim 1 or claim 2, wherein n is 0, or a salt of a compound of formula I.

**4.** A compound of formula I and, where applicable, tautomers thereof according to claim 1, wherein
$R_1$ is hydrogen or methyl;
$R_2$ is hydrogen or $C_1$-$C_4$alkyl;
$R_3$ is hydrogen, methyl or ethyl and
n is 0,
or a salt of a compound of formula I.

**5.** A compound according to claim 4 of formula

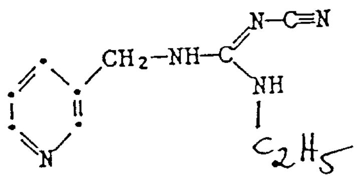

**6.** A process for the preparation of a compound of formula I or, where applicable, a tautomer thereof according to claim 1, or a salt of such a compound I, which comprises

a) reacting a compound of formula II

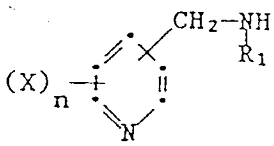

(II)

with a compound of formula III

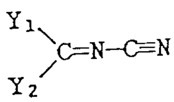

(III)

and with a compound of formula IV

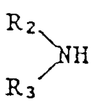

(IV);

or

b) for the preparation of a compound of formula I in which $R_2$ and $R_3$ are hydrogen, reacting a compound of formula II with dicyanimide;

wherein in the above formulae II to IV, $R_1$, $R_2$ and $R_3$, as well as X and n, have the meanings given in claim 1, and $Y_1$ and $Y_2$ are removable leaving groups;

and, if desired, a compound of formula I obtained according to a) or b) is converted in a manner known per se into one of its salts.

**7.** A process according to claim 6a) or 6b), which comprises reacting a compound of formula III with a compound of formula IV to form an intermediate of formula VI

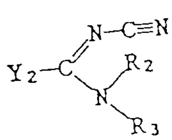

(VI)

and then reacting the resulting intermediate of formula VI with a compound of formula II, $R_1$, $R_2$, $R_3$, X,

n, $Y_1$ and $Y_2$ having the meanings given in claim 6.

**8.** The use of a compound of formula I or, where applicable, a tautomer thereof according to claim 1 or a salt of such a compound of formula I for controlling plant-attacking insects and representatives of the order Acarina.

**9.** Use according to claim 8 for controlling feeding insect pests in rice crops.

**10.** A composition for controlling plant-attacking insect pests and pests of the order Acarina comprising as active component at least one compound of formula I or, where applicable, a tautomer thereof according to claim 1, or a salt of such a compound I.

**11.** A method of controlling plant-attacking insects and representatives of the order Acarina, which comprises bringing into contact or treating the pests, or various stages of development thereof or the locus thereof, with a pesticidally effective amount of a compound of formula I or where applicable a tautomer thereof according to claim 1 or a salt of such a compound I, or with a composition according to claim 10 comprising a pesticidally effective amount of the active component together with adjuvants and carriers.

## Revendications

**1.** Composé de formule I et éventuellenent ses tautomères

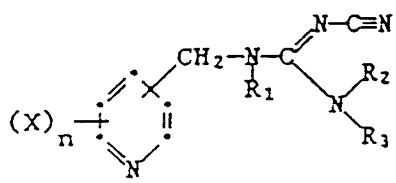

(I),

où

- $R_1$ représente l'hydrogène, un alkyle en $C_1$-$C_4$ ou un cyclopropyle;
- $R_2$ l'hydrogène, un alkyle en $C_1$-$C_4$, un cyclopropyle, le benzyle ou le picolyle;
- $R_3$ l'hydrogène, un alkyle en $C_1$-$C_4$ ou le cyclopropyle; ou $R_2$ et $R_3$ représentent ensemble $-(CH_2)_4-$ ou $-(CH_2)_5-$;
- X un halogène; et
- n est égal à 0, 1, 2 ou 3,

ainsi que les sels des composés de formule I; à l'exception de la N-picolyl-N-méthyl-N'-cyanoguanidine, de la N-picolyl-N'-méthyl-N''-cyanoguanidine, de la N-picolyl-N'-cyanoguanidine, de la N-(2-picolyl)-N'-t-butyl-N''-cyanoguanidine et de la N-(3-picolyl)-N'-t-butyl-N''-cyanoguanidine.

**2.** Composé de formule I et éventuellement ses tautomères selon la revendication 1, caractérisés en ce que

- $R_1$ et $R_3$ représentent l'hydrogène ou le méthyle;
- $R_2$ l'hydrogène, un alkyle en $C_1$-$C_4$ ou le benzyle; ou $R_2$ et $R_3$ représentent ensemble $-(CH_2)_5-$
- X le chlore et n est égal à 0, 1 ou 2, ainsi que les sels des composés de formule I.

**3.** Composé de formule I et éventuellement ses tautomères selon la revendication 1 ou 2, caractérisé en ce que n est égal à 0, ainsi que les sels des composés de formule I.

**4.** Composé de formule I et éventuellement ses tautomères selon la revendication 1, caractérisés en ce que

- $R_1$ représente l'hydrogène ou le méthyle;
- $R_2$ l'hydrogène ou un alkyle en $C_1$-$C_4$;
- $R_3$ l'hydrogène, le méthyle ou l'éthyle et n est égal à 0,

ainsi que les sels des composés de formule I.

**5.** Composés selon la revendication 4 de formule

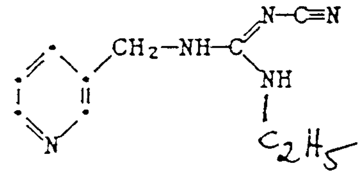 .

**6.** Procédé pour la préparation d'un composé de formule I ou éventuellement de l'un de ses tautomères selon la revendication 1 ou d'un sel d'un tel composé I, caractérisé

a) en ce que l'on fait réagir un composé de formule II

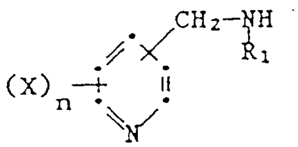 (II)

avec un composé de formule III

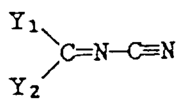 (III)

et avec un composé de formule IV

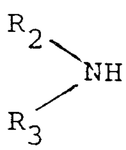 (IV)

ou

b) en ce que, pour la préparation d'un composé de formule I où $R_2$ et $R_3$ représentent l'hydrogène, l'on fait réagir un composé de formule II avec le dicyanimide;

$R_1$, $R_2$ et $R_3$ ainsi que X et n dans les formules II à IV précédentes ayant les significations données dans la revendication 1, et $Y_1$ et $Y_2$ représentant des groupes partants clivables;

et en ce que l'on transformer si désiré, d'une façon connue en soi, le composé de formule I obtenu selon a) ou b) en l'un de ses sels.

**7.** Procédé selon la revendication 6a) ou 6b), caractérisé en ce que l'on fait réagir un composé de formule III avec un composé de formule IV, conduisant à la formation d'un produit intermédiaire de formule VI

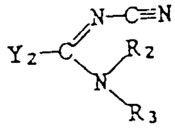 (VI)

et en ce que l'on fait réagir ensuite le produit intermédiaire de formule VI obtenu avec un composé de formule II, $R_1$, $R_2$, $R_3$, X, n, $Y_1$ et $Y_2$ ayant les significations données dans la revendication 6.

**8.** Utilisation d'un composé de formule I ou éventuellement de l'un de ses tautomères selon la revendication 1 ou d'un sel d'un tel composé de formule I pour lutter contre les insectes et représentants de l'ordre des acariens attaquant les plantes.

**9.** Utilisation selon la revendication 8 pour lutter contre les insectes nuisibles dévoreurs dans les cultures de riz.

**10.** Agent pour lutter contre les insectes nuisibles et les parasites de l'ordre des acariens attaquant les plantes, contenant comme composant actif au moins un composé de formule I ou éventuellement l'un de ses tautomères selon la revendication 1 ou un sel d'un tel composé I.

**11.** Procédé pour lutter contre les insectes et représentants de l'ordre des acariens attaquant les plantes, caractérisé en ce que l'on met en contact ou en ce que l'on traite les parasites ou leurs différents stades de développement ou les endroits où ils séjournent avec une quantité efficace comme pesticide d'un composé de formule I ou éventuellement de l'un de ses tautomères selon la revendication 1 ou d'un sel d'un tel composé I ou avec un agent selon la revendication 10, contenant à côté des additifs et supports une quantité efficace comme pesticide d'un composant actif.